# EUROPEAN PATENT APPLICATION

(11) **EP 4 685 742 A1**
(43) Date of publication of application: **28.01.2026**
(21) Application number: 24778081.0
(22) Date of filing: 27.03.2024
(51) Int. Cl.: G06T 7/13, G06T 7/00, G06T 17/20

(54) **TOOTH PREPARATION MARGIN LINE EXTRACTION METHOD AND APPARATUS, DEVICE, AND MEDIUM**

(30) Priority: 27.03.2023 CN 202310339615
(71) Applicant: Shining 3D Tech Co., Ltd., Hangzhou, Zhejiang 311258 (CN)
(72) Inventor: WANG, Jialei, Hangzhou, Zhejiang 311258 (CN); ZHANG, Jian, Hangzhou, Zhejiang 311258 (CN); JIANG, Tengfei, Hangzhou, Zhejiang 311258 (CN)
(74) Representative: Manitz Finsterwald Patent- und Rechtsanwaltspartnerschaft mbB
(86) International application number: PCT/CN2024/084163
(87) International publication number: WO 2024/199295

(57) **Abstract**

Embodiments of the present disclosure relate to a method, apparatus, device, and medium for extracting a cervical margin line of a tooth preparation, wherein the method includes: acquiring a three-dimensional digital model of the tooth preparation, and acquiring a candidate two-dimensional digital image corresponding to the three-dimensional digital model; determining a candidate cervical margin line according to the candidate two-dimensional digital image; and obtaining a target cervical margin line by performing error correction processing on the candidate cervical margin line in the three-dimensional digital model.

## Description

### CROSS-REFERENCE TO RELATED APPLICATIONS

The disclosure claims the priority of the Chinese patent application filed to the CNIPA on March 27, 2023, with the application number 2023103396156 and the invention title "Method, Apparatus, Device and Medium for Extracting Cervical Margin Line of Tooth Preparation", which is incorporated by reference in its entirety.

### TECHNICAL FIELD

The present disclosure generally relate to the technical field of three-dimensional digitization, more specifically, to a method, apparatus, device and medium for extracting the cervical margin line of a tooth preparation.

### BACKGROUND

In the fields related to oral medical care, the introduction of digital technologies has brought about profound changes. Extracting the cervical margin line of the tooth preparation is a key link in ensuring the marginal adaptation of oral restoration techniques. The accuracy of extracting cervical margin line may directly determine the shape of the final dental restoration. The tooth preparation refers to anatomical shape of a defective tooth that requires reconstruction and restoration; the cervical margin line of the tooth preparation refers to the outer edge line of the restoration model, which is extracted by dentists during the preliminary tooth model preparation step in oral restoration work. The cervical margin line of the tooth preparation is a closed curve including cervical margin features. It may have an important impact on comfort of the patient, oral health, and the success of oral restoration work that whether the cervical margin line of the tooth preparation fits the gingival part.

In related technologies, key points on the cervical margin line of the tooth preparation may be defined sequentially by technicians, and every two key points may be connected by using algorithms such as the shortest path. And finally, the cervical margin line may be obtained based on the results of connection.

However, the above method for obtaining the cervical margin line not only requires the algorithm to be robust, but also has certain requirements for skills of the operators. Additionally, it may consume a large amount of manual interaction and operation time, resulting in the inability to guarantee the efficiency and accuracy of obtaining the cervical margin line of the tooth preparation.

### SUMMARY

In order to address or at least partially solve the technical problems described above, the present disclosure provides a method, apparatus, device, and medium for extracting the cervical margin line of a tooth preparation. To address the above technical problems, embodiments of the present disclosure provide a method for extracting the cervical margin line of the tooth preparation, wherein the method may include: acquiring a three-dimensional digital model of the tooth preparation, and acquiring a candidate two-dimensional digital image corresponding to the three-dimensional digital model; determining a candidate cervical margin line based on the candidate two-dimensional digital image; and obtaining a target cervical margin line by performing error correction processing on the candidate cervical margin line in the three-dimensional digital model.

Embodiments of the present disclosure also provide an apparatus for extracting the cervical margin line of a tooth preparation, the apparatus may include: a first acquisition module configured to acquire a three-dimensional digital model of the tooth preparation; a second acquisition module configured to acquire a candidate two-dimensional digital image corresponding to the three-dimensional digital model; a determination module configured to determine a candidate cervical margin line according to the candidate two-dimensional digital image; and an error correction module configured to obtain a target cervical margin line by performing error correction processing on the candidate cervical margin line in the three-dimensional digital model.

The embodiments of the present disclosure also provide an electronic device. The electronic device may include: a processor; a memory for storing executable instructions of the processor; and the processor is configured to read the executable instructions from the memory and execute the instructions to implement the method for extracting the cervical margin line of the tooth preparation provided in the embodiments of the present disclosure.

The embodiments of the present disclosure also provide a computer-readable storage medium, wherein the storage medium stores a computer program, and the computer program is used to execute the method for extracting the cervical margin line of the tooth preparation provided in the embodiments of the present disclosure.

Compared with the related art, the technical solutions provided by the embodiments of the present disclosure have the following advantages:
The solution for extracting the cervical margin line of the tooth preparation provided by the embodiments of the present disclosure may obtain a three-dimensional digital model of the tooth preparation, acquire a candidate two-dimensional digital image corresponding to the three-dimensional digital model, then determine a candidate cervical margin line according to the candidate two-dimensional digital image, and obtain the target cervical margin line by performing error correction processing on the candidate cervical margin line in the three-dimensional digital model. In the embodiments of the present disclosure, the efficiency and accuracy of extracting the cervical margin line of the tooth preparation are improved.

It should be understood that the above general description and the following detailed description are only exemplary and explanatory, and cannot limit the present disclosure.

### BRIEF DESCRIPTION OF THE DRAWINGS

The above and other features, advantages, and aspects of various embodiments of the present disclosure will become more apparent with reference to the following detailed implementations in conjunction with the drawings. In the drawings, the same or similar reference numerals denote the same or similar elements. It should be understood that the drawings are schematic, and the components and elements are not necessarily drawn to scale.
FIG. 1 is a schematic flowchart of a method for extracting a cervical margin line of a tooth preparation provided by some embodiments of the present disclosure;
FIG. 2 is a schematic diagram of expanding a three-dimensional digital model into a candidate two-dimensional digital image provided by some embodiments of the present disclosure;
FIG. 3 is a schematic diagram of a candidate cervical margin line provided by some embodiments of the present disclosure;
FIG. 4 is a schematic diagram of a three-dimensional digital model provided by some embodiments of the present disclosure;
FIG. 5 is a schematic diagram of a candidate cervical margin line and a target cervical margin line provided by some embodiments of the present disclosure;
FIG. 6 is a schematic diagram of a scenario for extracting a cervical margin line of a tooth preparation provided by some embodiments of the present disclosure;
FIG. 7 is a structural schematic diagram of an apparatus for extracting a cervical margin line of a tooth preparation provided by some embodiments of the present disclosure;
FIG. 8 is a structural schematic diagram of an electronic device provided by some embodiments of the present disclosure.

### DETAILED DESCRIPTION

The following will describe the embodiments of the present disclosure with more details with reference to the drawings. Although the drawings show some certain embodiments of the present disclosure, it should be understood that the present disclosure may be implemented in various forms and should not be construed as being limited to the embodiments herein. On the contrary, the embodiments are provided for more thorough and complete understanding of the present disclosure. It should be understood that the drawings and embodiments of the present disclosure are only for illustrative purposes and are not intended to limit the protection scope of the present disclosure.

It should be understood that the various steps described in method implementations of the present disclosure may be executed in different orders and/or in parallel. In addition, the method implementations may include additional steps and/or omit the execution of the illustrated steps. The scope of the present disclosure is not limited in this regard.

The term "including" and its variants as used herein are open-ended inclusions, which means that "including but not limited to". The term "based on" means "based at least in part on". The term "one embodiment" means "at least one embodiment"; the term "another embodiment" means "at least one additional embodiment"; the term "some embodiments" means "at least some embodiments". Relevant definitions of other terms will be given in the following description.

It should be noted that the concepts such as "first" and "second" mentioned in the present disclosure are only used to distinguish different apparatus, modules or units, and are not used to limit the order of functions performed by the apparatus, modules or units or their interdependencies.

It should be noted that the modifications of "a" and "a plurality of" mentioned in the present disclosure are illustrative rather than restrictive. Those skilled in the art should understand that unless clearly indicated otherwise in the context, they should be understood as "one or more".

The names of messages or information interacted between multiple apparatus in the implementations of the present disclosure are only for illustrative purposes, and are not used to limit the scope of the messages or information.

In order to solve the above problem, the embodiments of the present disclosure provide a method for extracting a cervical margin line of a tooth preparation. The method, on one hand, may determine initially the cervical margin line of the tooth preparation in a two-dimensional digital image. When identifying the cervical margin line in the two-dimensional digital image, the recognition efficiency may be high, and it will not be interfered by the diversity of preparations and the quality of grid surfaces, which initially ensures the accuracy and efficiency of extracting the cervical margin line. On the other hand, the method may map the initially determined cervical margin line of the tooth preparation to the digital three-dimensional model, so as to ensure that the cervical margin line may be further refined in the digital three-dimensional model. Thus, the final cervical margin line after refined extraction may be obtained, which guarantees the accuracy of the final cervical margin line.

The method may be introduced below in combination with specific embodiments.

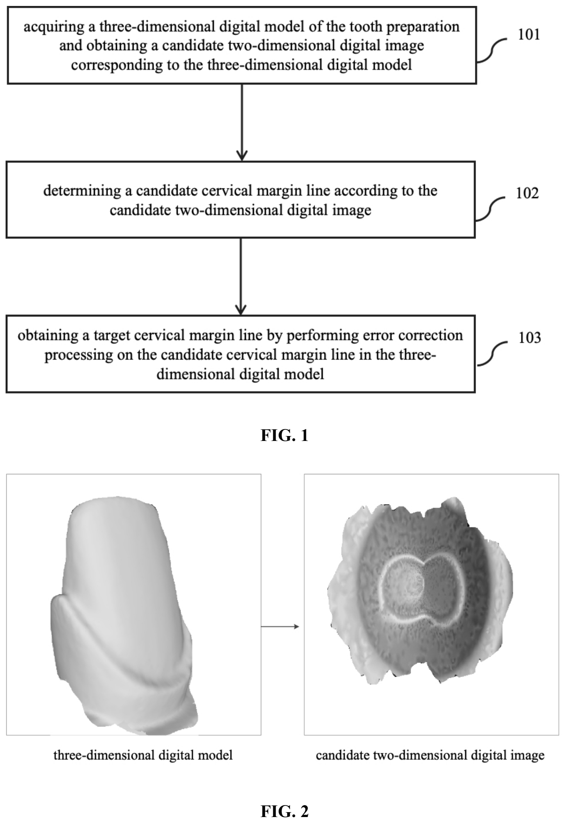

FIG. 1 is a schematic flowchart of a method for extracting the cervical margin line of a tooth preparation provided by some embodiments of the present disclosure. The method may be executed by an apparatus for extracting the cervical margin line of the tooth preparation, wherein the apparatus may be implemented by software and/or hardware, and may generally be integrated into an electronic device. As shown in FIG. 1, the method includes following steps.

Step 101: acquiring a three-dimensional digital model of the tooth preparation, and obtaining a candidate two-dimensional digital image corresponding to the three-dimensional digital model.

In an embodiment of the present disclosure, the three-dimensional digital model of the tooth preparation from which the cervical margin line is to be extracted may be acquired. In some possible implementations, the three-dimensional digital model of the tooth preparation may be obtained by scanning a target oral cavity using a preset three-dimensional data scanning device, wherein the preset three-dimensional data scanning device may be intraoral scanner with high-precision or the like. The intraoral scanner may use a probing optical scanning head to directly scan the interior of the oral cavity of the patient. Based on the principle of structured light triangulation imaging, a digital projection system may be used to project light patterns onto the tooth preparation and other structures. And then a camera acquisition system may capture the pattern and obtain the three-dimensional digital model by performing three-dimensional reconstruction and stitching through algorithm processing. The three-dimensional digital model may be composed of curved surface patches of multiple triangular meshes.

Considering that directly extracting the cervical margin line from the three-dimensional digital model may be affected by geometric characteristics of the three-dimensional digital model, resulting in low robustness of the extraction algorithm and affecting the efficiency and accuracy of extracting the cervical margin line. While the recognition algorithm in the two-dimensional field may have relatively high accuracy and strong robustness. Thus, in the embodiments of the present disclosure, the candidate two-dimensional digital image corresponding to the three-dimensional digital model may be acquired.

In some embodiments, for different application scenarios, the ways to expand the three-dimensional digital model to obtain candidate two-dimensional digital images may be different. The examples may be as follows.

In some possible examples, the mesh vertices in the corresponding triangular meshes of the three-dimensional digital model may be determined, and the candidate two-dimensional digital images may be obtained based on the mesh vertices.

For example, first two-dimensional pixel points corresponding to each mesh vertex in the three-dimensional digital model may be determined based on a first preset algorithm. The first preset algorithm may include but is not limited to Least Square Conformal Maps (LSCM) algorithm. The LSCM algorithm may ensure that the local scale and feature information may be preserved as much as possible after the three-dimensional mesh is converted to a two-dimensional plane. Based on the LSCM algorithm, the first two-dimensional pixel points (u, v) on the two-dimensional plane corresponding to each mesh vertex (x, y, z) may be obtained

Since the three-dimensional digital model may include not only the mesh vertices in the triangular meshes but also the three-dimensional points inside the triangles. In the embodiments of the present disclosure, a first curvature value of each mesh vertex is also obtained. Second two-dimensional pixel points corresponding to internal feature points in each triangular mesh of the three-dimensional digital model may be determined according to a preset interpolation algorithm and the curvature values. That is, the curvature information on the vertices of the three-dimensional mesh may be converted to the two-dimensional RGB space (0-255), and the pixel values are assigned to each second two-dimensional pixel point on the two-dimensional plane through the interpolation method corresponding to the preset interpolation algorithm, thus forming the candidate two-dimensional digital image.

In an embodiment of the present disclosure, the corresponding candidate two-dimensional digital image may be generated by performing mesh parameterization based on the three-dimensional digital model. That is, the first preset algorithm is mesh parameterization, and preferably, the mesh parameterization is LSCM algorithm.

For example, as shown in FIG. 2, through the first preset algorithms such as mesh parameterization described above, the corresponding candidate two-dimensional digital image may may be obtained by expanding the three-dimensional digital model, wherein the two-dimensional pixel points in the candidate two-dimensional digital image may correspond to the corresponding three-dimensional model points in the three-dimensional digital model. That is, while the corresponding candidate two-dimensional digital image may be generated by performing mesh parameterization on the three-dimensional digital model, a mapping relationship between the two-dimensional pixel points in the candidate two-dimensional digital image and the three-dimensional model points in the three-dimensional digital model is formed. One two-dimensional pixel point in the candidate two-dimensional digital image may determine a corresponding three-dimensional model point in the three-dimensional digital model, and one three-dimensional model point in the three-dimensional digital model may determine a corresponding two-dimensional pixel point in the candidate two-dimensional digital image.

In some possible examples, the coordinate conversion relationship between the three-dimensional model points in the three-dimensional digital model and the two-dimensional pixel points in the candidate two-dimensional digital image in the present embodiment may be predetermined. For example, multiple sets of sample data may be constructed in advance, wherein each set of sample data may include a sample three-dimensional digital model and a sample two-dimensional digital image corresponding to the sample three-dimensional digital model. Multiple reference pixel points in each sample two-dimensional digital image may be obtained, and multiple reference three-dimensional points, which may match the multiple reference pixel points, may be determined in the corresponding sample three-dimensional digital model, wherein the reference pixel points and corresponding reference three-dimensional points may refer to the same point on the tooth, and the matching method of multiple reference pixel points and reference three-dimensional points may be realized by feature matching algorithms, etc.

Furthermore, the two-dimensional coordinates of each reference pixel point and the three-dimensional coordinates of the corresponding reference three-dimensional points may be obtained, and the coordinate conversion relationship may be determined based on the two-dimensional coordinates and the three-dimensional coordinates.

In the present example, the two-dimensional pixel point corresponding to the three-dimensional coordinate point of the three-dimensional digital model may be determined based on the coordinate conversion relationship, and further the candidate two-dimensional digital image composed of two-dimensional pixel points may be obtained.

Step 102: determining a candidate cervical margin line according to the candidate two-dimensional digital image.

In an embodiment of the present disclosure, the candidate cervical margin line may be determined based on the candidate two-dimensional digital image. As a result, since the algorithms of determining the candidate cervical margin line in the candidate two-dimensional digital image may have strong robustness, The efficiency and accuracy of extracting the candidate cervical margin line are relatively high

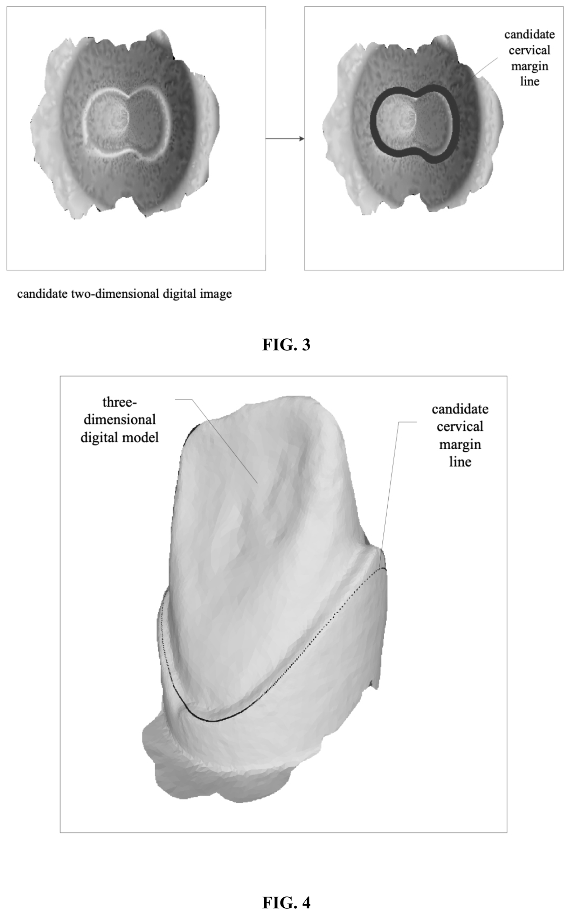

In some optional implementation, a deep learning model may be trained using a large amount of sample data, wherein the sample data includes two-dimensional digital images and manually marked cervical margin lines. The specific training process may include: inputting the two-dimensional digital images without marked cervical margin lines into the deep learning model, outputting an area mask of the cervical margin line, comparing the mask with the area of the manually marked cervical margin line to calculate the loss function, then optimizing the deep learning model through a backpropagation algorithm, and repeating the above process iteratively until the loss function no longer decreases, which may indicate that the deep learning model has converged, and obtaining the trained deep learning model. A "predicted" area of the cervical margin line may be output by inputting the candidate two-dimensional digital image into the pre-trained deep learning model, that is, the candidate cervical margin line may be determined. Taking the scenario shown in FIG. 3 as an example, the candidate two-dimensional digital image obtained in FIG. 2 may be input into the pre-trained deep learning model, and the candidate cervical margin line output by the model may be quickly obtained.

Further, considering that the candidate two-dimensional digital image may not completely correspond to the three-dimensional digital model, for example, in the case that the second two-dimensional pixel points in the three-dimensional digital model are obtained by interpolating the first curvature values of each mesh vertex according to a preset interpolation algorithm, the second two-dimensional pixel points may have certain errors, which may result that the candidate cervical margin line may also have errors. Therefore, in order to further refine the candidate cervical margin line, the candidate cervical margin line may also be determined in the three-dimensional digital model, so as to facilitate further refinement of the candidate cervical margin line in the three-dimensional digital model.

It should be noted that, for different application scenarios, the ways to determine the two-dimensional candidate cervical margin line in the three-dimensional digital model are different, with the following examples.

In some possible embodiments, the candidate cervical margin line is marked in the candidate two-dimensional digital image to obtain a target two-dimensional digital image, wherein the candidate cervical margin line in the target two-dimensional digital image is a two-dimensional candidate cervical margin line. The target two-dimensional digital image may be reprojected into the three-dimensional digital model to determine the candidate cervical margin line corresponding to the two-dimensional candidate cervical margin line in the three-dimensional digital model, wherein the candidate cervical margin line in the three-dimensional digital model is a three-dimensional candidate cervical margin line. Reprojection refers to determining the three-dimensional candidate cervical margin line in the three-dimensional digital model corresponding to the two-dimensional candidate cervical margin line in the target two-dimensional digital image. It should be noted that the target two-dimensional digital image is a candidate two-dimensional digital image with a two-dimensional candidate cervical margin line determined, and there is a mapping relationship between the two-dimensional pixel points in the candidate two-dimensional digital image and the three-dimensional model points in the three-dimensional digital model, which is also the mapping relationship between the two-dimensional pixel points in the target two-dimensional digital image and the three-dimensional model points in the three-dimensional digital model. Based on the mapping relationship, the three-dimensional candidate cervical margin line corresponding to the two-dimensional candidate cervical margin line in the target two-dimensional digital image may naturally be determined in the three-dimensional digital model. The two-dimensional candidate cervical margin line may be composed of a plurality of two-dimensional pixel points corresponding to the candidate cervical margin line in the two-dimensional digital image, and the three-dimensional candidate cervical margin line may be composed of a plurality of three-dimensional model points corresponding to the three-dimensional candidate cervical margin line in the three-dimensional digital model. The reprojection in the present embodiment may also be implemented using any reprojection algorithm in the existing technology, including but not limited to the grid reprojection algorithm, etc.

When reprojecting the target two-dimensional digital image into the three-dimensional digital model, the mapping relationship between the two-dimensional pixel points in the target two-dimensional digital image and corresponding three-dimensional model points in the three-dimensional digital model is determined. The mapping relationship may describe a correspondence between the two-dimensional pixel points and three-dimensional model points of one same point on the tooth preparation. For example, the mapping relationship may be coordinate conversion relationship between the two-dimensional pixel points and three-dimensional model points of the same point on the tooth preparation.

Furthermore, a first three-dimensional points in the three-dimensional digital model corresponding to the two-dimensional points in the two-dimensional candidate cervical margin line may be determined according to the mapping relationship. For example, sampled two-dimensional points may be determined in the two-dimensional candidate cervical margin line using a random sampling algorithm, and the first three-dimensional points corresponding to the sampled two-dimensional points may be determined based on the mapping relationship; alternatively, the first three-dimensional points in the three-dimensional digital model corresponding to each two-dimensional point in the two-dimensional candidate cervical margin line may be determined according to the mapping relationship. And then, the candidate cervical margin line in the three-dimensional digital model may be obtained based on the first three-dimensional points.

It should be emphasized that the purpose of determining the three-dimensional candidate cervical margin line corresponding to the two-dimensional candidate cervical margin line in the three-dimensional digital model as mentioned above is to determine a position of the candidate cervical margin line in the three-dimensional digital model, facilitating further refinement of the candidate cervical margin line in the three-dimensional digital model. The three-dimensional candidate cervical margin line corresponding to the two-dimensional candidate cervical margin line in the three-dimensional digital model may be either non-visual or visual. In some possible implementations, in order to enhance intuitiveness of obtaining the cervical margin line of the tooth preparation, the three-dimensional candidate cervical margin line corresponding to the two-dimensional candidate cervical margin line in the three-dimensional digital model is displayed in a visual way. For example, as shown in FIG. 4, the three-dimensional digital model is displayed, and the corresponding candidate cervical margin line is shown in the three-dimensional digital model.

Step 103: obtaining the target cervical margin line by performing error correction processing on the candidate cervical margin line in the three-dimensional digital model.

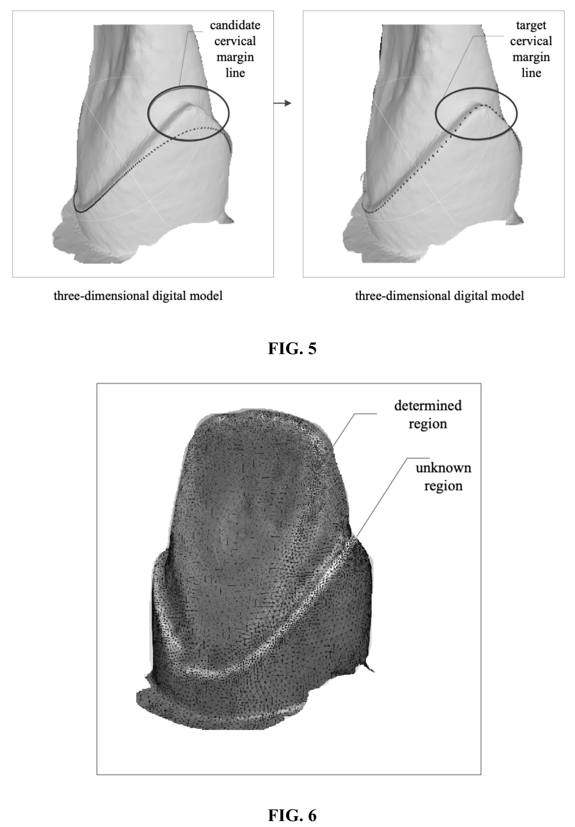

In an embodiment of the present disclosure, after obtaining the three-dimensional digital model, the target cervical margin line may be obtained by performing error correction processing on the candidate cervical margin line in the three-dimensional digital model. As shown in FIG. 5, the target cervical margin line corrects the errors of the candidate cervical margin line, ensuring the accuracy of the target cervical margin line.

It should be noted that, for different application scenarios, the ways of performing error correction processing on the candidate cervical margin line in the three-dimensional digital model to obtain the target cervical margin line may be different, with the following examples.

In some possible embodiments, some algorithms, such as the random walk algorithm, may be used for error correction processing. In the present embodiment, a determined region and an unknown region in the three-dimensional digital model may be determined according to the candidate cervical margin line, wherein the determined region may be a region composed of three-dimensional points that are confirmed not to belong to the cervical margin line, which may be usually located in the cusp region (the upper half region of the tooth preparation as shown in FIG. 6). The unknown region may be a region composed of three-dimensional points that may belong to the candidate cervical margin line and the nearby three-dimensional points (the candidate cervical margin line and its nearby region as shown in FIG. 6).

In some possible embodiments, in the three-dimensional digital model, associated three-dimensional points of the second three-dimensional points in the candidate cervical margin line may be determined. For example, the associated three-dimensional points of the sampled second three-dimensional points in the candidate cervical margin line may be determined; alternatively, the associated three-dimensional points of each second three-dimensional point in the candidate cervical margin line may be determined. The associated three-dimensional points may be understood as three-dimensional points in the three-dimensional digital model that are at a distance from the corresponding second three-dimensional points in the candidate cervical margin line, which is less than a preset distance threshold, wherein the preset distance threshold may be calibrated based on experimental data. In the present embodiment, the region corresponding to the second two-dimensional points and the associated three-dimensional points may be determined as the unknown region. That is, in the present embodiment, the candidate cervical margin line and its surrounding region may be taken as the unknown region according to a preset distance. In the present embodiment, in order to ensure the accuracy of determining the unknown region, the long axis of the tooth in the three-dimensional digital model of the tooth preparation is parallel to the Z-axis in the coordinate system (it should be emphasized that "parallel" may be understood as the direction of central axis of the long axis of the tooth being parallel to the Z-axis, and visually, the long axis of the tooth may be roughly parallel to the Z-axis), wherein the direction from the root to the crown in the three-dimensional digital model of the tooth preparation is the positive direction of the Z-axis. It should be noted that in the present disclosure, the first three-dimensional points, the second three-dimensional points, three-dimensional model points, etc., may be all three-dimensional points containing three-dimensional coordinate information in the three-dimensional digital model.

Further, in the present embodiment, according to a second preset algorithm, the second curvature values of the sampled mesh points in the three-dimensional digital model may be traversed from the determined region to the unknown region, wherein the sampled mesh points may be any three-dimensional point belonging to the target tooth model, and the second preset algorithm may be the random walk algorithm, etc. described above.

Target sampled mesh points that meet the preset convergence conditions corresponding to the second preset algorithm may be determined according to the second curvature values. When the second preset algorithm is the random walk algorithm, the logic of the random walk algorithm is as follows: starting traversing a graph from one or a series of vertices; at any vertex, the traverser may walk to neighbor vertices of the current vertex with a probability of 1-a, and randomly jump to any vertex in the graph with a probability of a (the vertices jumped may be the sampled mesh points described above), wherein a may be called a jump probability. After each walk, a probability distribution may be obtained, which may describe probabilities that each vertex in the graph is accessed. The probability distribution may be used as the input for the next walk, and the process may be iterated repeatedly. When the preset convergence conditions are met, the probability distribution will tend to converge.

In the present embodiment, considering that the second curvature value of the sampled mesh points on the cervical margin line is greater than the curvature value of the sampled mesh points not on the cervical margin line, the convergence condition in the present embodiment is used to address the problem that the target sampled mesh points on the target cervical margin line cannot be accurately determined when walking near the cervical margin line. In the present embodiment, the preset convergence condition may be that the second curvature value of the current sampled mesh point is greater than the second curvature value of the previous adjacent sampled mesh point, and the second curvature value of the current sampled mesh point is greater than the second curvature value of the next adjacent sampled mesh point. That is, in the case that the current sampled mesh point has the second curvature value that may be greater than the second curvature value of adjacent sampled mesh points, it may be considered that the preset convergence condition is met, and the random walk is stopped to determine that the current sampled mesh point is the target sampled mesh point.

In the actual execution process, in order to further ensure that the target sampled mesh points belong to the cervical margin line, when traversing the second curvature values of the sampled mesh points in the three-dimensional digital model according to the second preset algorithm, the second curvature values of the sampled mesh points with larger second curvature values may be further increased. For example, the initial curvature values of the mesh points in the unknown region may be identified, and it may be determined that whether the initial curvature values are greater than or equal to a preset curvature threshold (wherein the preset curvature threshold may be set according to scene requirements). In the case that the initial curvature values are greater than the preset curvature threshold, it may increase the weight of the initial curvature values. For example, weighted curvature values may be obtained by multiplying the initial curvature values greater than the preset curvature threshold by a preset weight value greater than 1. Thus, it may be further ensured that when the random walk algorithm walks to the target sampled mesh points, the walk may be stopped, ensuring the accuracy of the target sampled mesh points.

Further, after determining the target sampled mesh points, the target cervical margin line may be obtaining by performing fitting processing on the target sampled mesh points. The fitting algorithms for fitting the target sampled mesh points may include, but are not limited to, Bézier curve fitting, three-dimensional B-spline fitting, and the like. In some possible embodiments, in order to prevent the target cervical margin line from being insufficiently smooth, a fairing method may be used to process the target sampled mesh points to obtain the target cervical margin line. That is, after obtaining a fitting curve by fitting the target sampled mesh points according to a preset fitting algorithm, multiple sampled three-dimensional points may be obtained by sampling the fitting curve. The sampled three-dimensional points may be obtained by any random sampling algorithm. Further, the corresponding target cervical margin line may be obtained based on the multiple sampled three-dimensional points. That is, in the present embodiment, a smoother target cervical margin line may be obtained by re-sampling and fitting the fitting curve

In some possible embodiments, since the curvature values near the cervical margin line are significantly larger, after determining the candidate cervical margin line, the third curvature values of the candidate cervical margin three-dimensional points that belong to vertices of triangular meshes in each candidate cervical margin line may be also determined, and the fourth curvature values of other adjacent three-dimensional mesh vertices on the triangular mesh where each candidate cervical margin three-dimensional point is located may be determined. In the case that the third curvature value is greater than the fourth curvature values of all adjacent other three-dimensional mesh points, the candidate cervical margin three-dimensional point may be determined as a target cervical margin three-dimensional point on the target cervical margin line. In the case that the third curvature value is not greater than the fourth curvature values of adjacent other three-dimensional mesh vertices, the maximum fourth curvature value among the fourth curvature values of the adjacent other three-dimensional mesh vertices may be determined, and then the maximum fourth curvature value may be compared with the fifth curvature values of other adjacent three-dimensional mesh vertices on the triangular mesh where it is located. In the case that the fourth curvature value is greater than the fifth curvature values of all adjacent other three-dimensional mesh points, the three-dimensional mesh vertex corresponding to the fourth curvature value may be determined as the target cervical margin three-dimensional point on the target cervical margin line. In the case that the fourth curvature value is not greater than the fifth curvature values of adjacent other three-dimensional mesh vertices, the maximum fifth curvature value among the fifth curvature values of the adjacent other three-dimensional mesh vertices may be determined, and then the maximum fifth curvature value may be compared with the sixth curvature values of other adjacent three-dimensional mesh vertices on the triangular mesh where it is located... The cycle may be continued to determine multiple target cervical margin three-dimensional points belonging to the target cervical margin line, and the target cervical margin line may be determined based on the target cervical margin three-dimensional points.

The method for extracting the cervical margin line of the tooth preparation provided in the present disclosure may improve the efficiency and accuracy of extracting the cervical margin line of the tooth preparation, and has strong industrial practicability.

In summary, the method for extracting the cervical margin line of the tooth preparation according to the embodiments of the present disclosure may obtain the three-dimensional digital model of the tooth preparation, acquire the candidate two-dimensional digital digital image corresponding to the three-dimensional digital model, then determine the candidate cervical margin line based on the candidate two-dimensional digital image, and obtain the target cervical margin line by performing error correction processing on the candidate cervical margin line in the three-dimensional digital model. In the embodiments of the present disclosure, the efficiency and accuracy of extracting the cervical margin line of the tooth preparation may be improved.

In order to implement the above embodiments, the present disclosure also provides an apparatus for extracting the cervical margin line of the tooth preparation.

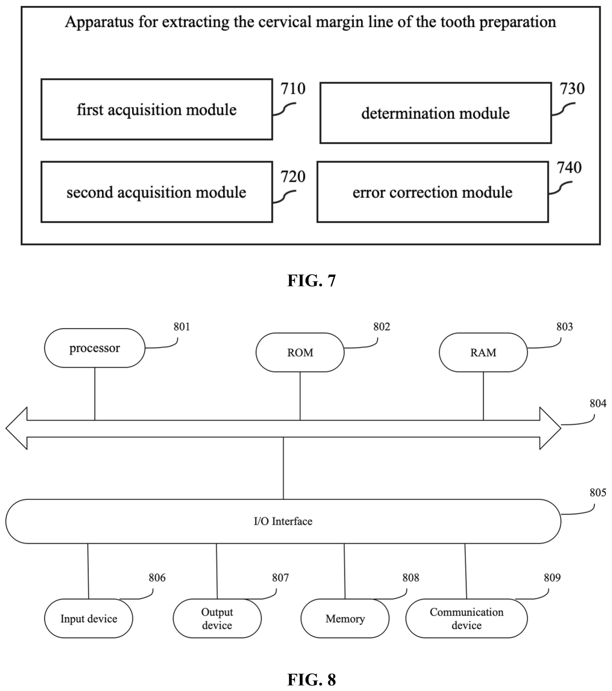

FIG. 7 is a schematic structural diagram of an apparatus for extracting a cervical margin line of a tooth preparation provided by some embodiments of the present disclosure. The apparatus may be implemented by software and/or hardware, and may generally be integrated into an electronic device to extract the cervical margin line of the tooth preparation. As shown in FIG. 7, the apparatus may include: a first acquisition module 710, a second acquisition module 720, a determination module 730, and an error correction module 740.

The first acquisition module 710 is configured to acquire a three-dimensional digital model of the tooth preparation.

The second acquisition module 720 is configured to acquire a candidate two-dimensional digital image corresponding to the three-dimensional digital model.

The determination module 730 is configured to determine a candidate cervical margin line according to the candidate two-dimensional digital image.

The error correction module 740 is configured to obtain the target cervical margin line by performing error correction processing on the candidate cervical margin line in the three-dimensional digital model.

The apparatus for extracting the cervical margin line of the tooth preparation provided by the embodiment of the present disclosure may execute the method for extracting the cervical margin line of the tooth preparation provided by any embodiment of the present disclosure, and include corresponding functional modules and beneficial effects for executing the method. The implementation principle is similar, and details are not repeated here.

In order to implement the above embodiments, the present disclosure also provides a computer program product, including computer programs or instructions, which when executed by a processor, implement the method for extracting the cervical margin line of the tooth preparation in the above embodiments.

FIG. 8 is a schematic structural diagram of an electronic device provided by some embodiments of the present disclosure.

With specific reference to FIG. 8 below, it shows a structural schematic diagram suitable for implementing the electronic device 800 in the embodiments of the present disclosure. The electronic device 800 in the embodiments of the present disclosure may include, but is not limited to, mobile terminals such as mobile phones, notebook computers, digital broadcast receivers, Personal Digital Assistants (PDAs), Tablet Computers (PADs), Portable Multimedia Players (PMPs), vehicle-mounted terminals (such as vehicle-mounted navigation terminals), etc., and fixed terminals such as digital TVs, desktop computers, etc. The electronic device shown in FIG. 8 is only an example, and should not impose any limitation on the functions and scope of use of the embodiments of the present disclosure.

As shown in FIG. 8, the electronic device 800 may include a processor 801 (such as a central processing unit, a graphics processor, etc.), which may execute various suitable actions and processes according to programs stored in a read-only memory (ROM) 802 or programs loaded from a memory 808 into a random access memory (RAM) 803. The RAM 803 may also store various programs and data required for the operation of the electronic device 800. The processor 801, ROM 802, and RAM 803 are connected to each other through a bus 804. An input/output (I/O) interface 805 is also connected to the bus 804

In general, the following devices may be connected to the I/O interface 805: input devices 806 including, for example, a touch screen, a touchpad, a keyboard, a mouse, a camera, a microphone, an accelerometer, a gyroscope, etc.; output devices 807 including, for example, a liquid crystal display (LCD), a speaker, a vibrator, etc.; memory 808 including, for example, a magnetic tape, a hard disk, etc.; and a communication device 809. The communication device 809 may allow the electronic device 800 to perform wireless or wired communication with other devices to exchange data. Although FIG. 8 shows the electronic device 800 with various devices, it should be understood that it is not required to implement or have all the shown devices. Instead, more or fewer devices may be implemented or provided.

In particular, according to the embodiments of the present disclosure, the processes described above with reference to the flowcharts may be implemented as computer software programs. For example, the embodiments of the present disclosure include a computer program product, which includes a computer program carried on a non-transitory computer-readable medium, and the computer program includes program code for executing the method shown in the flowchart. In such embodiments, the computer program may be downloaded and installed from the network through the communication device 809, or installed from the memory 808, or installed from the ROM 802. When the computer program is executed by the processor 801, the above-mentioned functions defined in the method for extracting the cervical margin line of the tooth preparation according to the embodiments of the present disclosure are executed.

It should be noted that the above-mentioned computer-readable medium in the present disclosure may be a computer-readable signal medium, a computer-readable storage medium, or any combination of the two. The computer-readable storage medium may be, for example, but not limited to, an electrical, magnetic, optical, electromagnetic, infrared, or semiconductor system, apparatus, or device, or any combination of the above. More specific examples of computer-readable storage media may include, but are not limited to: an electrical connection with one or more wires, a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage device, a magnetic storage device, or any suitable combination of the above. In the present disclosure, the computer-readable storage medium may be any tangible medium that includes or stores a program, which may be used by or in combination with an instruction execution system, apparatus, or device. In the present disclosure, the computer-readable signal medium may include a data signal propagated in baseband or as part of a carrier wave, in which computer-readable program code is carried. Such a propagated data signal may take various forms, including but not limited to electromagnetic signals, optical signals, or any suitable combination of the above. A computer-readable signal medium may also be any computer-readable medium other than a computer-readable storage medium, which may send, propagate, or transmit a program for use by or in combination with an instruction execution system, apparatus, or device. The program code contained on the computer-readable medium may be transmitted using any appropriate medium, including but not limited to: electric wires, optical cables, RF (radio frequency), etc., or any suitable combination of the above.

In some implementations, clients and servers may communicate using any currently known or future-developed network protocols such as HyperText Transfer Protocol (HTTP), and may be interconnected with digital data communication (for example, communication networks) in any form or medium. Examples of communication networks include local area networks ("LAN"), wide area networks ("WAN"), the Internet (for example, the Internet), and peer-to-peer networks (for example, ad hoc peer-to-peer networks), as well as any currently known or future-developed networks.

The above-mentioned computer-readable medium may be included in the above-mentioned electronic device; it may also exist independently without being assembled into the electronic device.

The above-mentioned computer-readable medium carries one or more programs, and when the above-mentioned one or more programs are executed by the electronic device, the electronic device may be enabled to: acquire a three-dimensional digital model of the tooth preparation, acquire a candidate two-dimensional digital image corresponding to the three-dimensional digital model, then determine a candidate cervical margin line according to the candidate two-dimensional digital image, and obtain the target cervical margin line by performing error correction processing on the candidate cervical margin line in the three-dimensional digital model. In the embodiments of the present disclosure, the efficiency and accuracy of extracting the cervical margin line of the tooth preparation are improved.

The electric device may write computer program code for performing operations of the present disclosure in one or more programming languages or combinations thereof, including but not limited to object-oriented programming languages such as Java, Smalltalk, and C++, as well as conventional procedural programming languages such as the "C" language or similar programming languages. The program code may execute entirely on the computer of the user, partly on the computer of the user, as a stand-alone software package, partly on the computer of the user and partly on a remote computer, or entirely on the remote computer or server. In the cases involving a remote computer, the remote computer may be connected to the computer of the user through any type of network, including a local area network (LAN) or a wide area network (WAN), or may be connected to an external computer (for example, through an Internet connection using an Internet service provider).

The flowcharts and block diagrams in the drawings may illustrate the possible architectures, functions, and operations of systems, methods, and computer program products according to various embodiments of the present disclosure. In this regard, each block in the flowcharts or block diagrams may represent a module, program segment, or portion of code that includes one or more executable instructions for implementing the specified logical function. It should also be noted that, in some alternative implementations, the functions noted in the blocks may occur in a different order than that noted in the drawings. For example, two blocks consecutively represented may actually execute substantially in parallel, or the two blocks may sometimes execute in the reverse order, which may depend on the functions involved. It should also be noted that each block in the block diagrams and/or flowcharts, and combinations of blocks in the block diagrams and/or flowcharts, may be implemented by special-purpose hardware-based systems that perform the specified functions or operations, or by combinations of special-purpose hardware and computer instructions.

The units involved in the embodiments described in the present disclosure may be implemented by means of software or hardware. In some cases, the name of a unit does not constitute a limitation on the unit itself.

The functions described above in the present disclosure may be performed, at least in part, by one or more hardware logic components. For example, without limitation, exemplary types of hardware logic components that may be used include: Field-Programmable Gate Arrays (FPGA), Application-Specific Integrated Circuits (ASIC), Application-Specific Standard Products (ASSP), System-on-Chip (SOC), Complex Programmable Logic Devices (CPLD), and so on.

In the context of the present disclosure, a machine-readable medium may be a tangible medium that may include or store a program for use by or in conjunction with an instruction execution system, apparatus, or device. A machine-readable medium may be a machine-readable signal medium or a machine-readable storage medium. Machine-readable media may include, but are not limited to, electronic, magnetic, optical, electromagnetic, infrared, or semiconductor systems, devices, or equipment, or any suitable combination of the foregoing. More specific examples of machine-readable storage media would include electrical connections based on one or more wires, portable computer disks, hard disks, Random Access Memory (RAM), Read-Only Memory (ROM), Erasable Programmable Read-Only Memory (EPROM or flash memory), optical fibers, portable compact disk read-only memory (CD-ROM), optical storage devices, magnetic storage devices, or any suitable combination of the foregoing.

The above description is merely a preferred embodiment of the present disclosure and an explanation of the technical principles applied. Those skilled in the art should understand that the scope of disclosure involved in the present disclosure is not limited to the technical solutions formed by the specific combination of the above technical features, but also should cover other technical solutions formed by any combination of the above technical features or their equivalent features without departing from the above disclosed concept. For example, technical solutions formed by replacing the above features with technical features disclosed in the present disclosure (but not limited to) that have similar functions.

In addition, although the operations are depicted in a specific order, this should not be construed as requiring these operations to be performed in the specific order shown or in a sequential order. Under certain circumstances, multitasking and parallel processing may be advantageous. Similarly, although several specific implementation details are included in the above discussion, these should not be interpreted as limiting the scope of the present disclosure. Certain features described in the context of separate embodiments can also be implemented in combination in a single embodiment. Conversely, various features described in the context of a single embodiment can also be implemented in multiple embodiments individually or in any suitable subcombination.

Although the subject matter has been described using language specific to structural features and/or methodological logical actions, it should be understood that the subject matter defined in the appended claims is not necessarily limited to the specific features or actions described above. On the contrary, the specific features and actions described above are merely example forms of implementing the claims

## Claims

1. A method for extracting a cervical margin line of a tooth preparation, comprising:
acquiring a three-dimensional digital model of the tooth preparation and acquiring a candidate two-dimensional digital image corresponding to the three-dimensional digital model;
determining a candidate cervical margin line according to the candidate two-dimensional digital image;
obtaining a target cervical margin line by performing error correction processing on the candidate cervical margin line in the three-dimensional digital model.

2. The method according to claim 1, wherein obtaining the candidate two-dimensional digital image corresponding to the three-dimensional digital model comprises:
determining mesh vertices in a triangular mesh of the three-dimensional digital model;
obtaining the candidate two-dimensional digital image according to the mesh vertices.

3. The method according to claim 1 or claim 2, wherein obtaining the candidate two-dimensional digital image according to the mesh vertices comprises:
determining first two-dimensional pixel points corresponding to the mesh vertices according to a first preset algorithm;
obtaining first curvature values of the mesh vertices, and determining second two-dimensional pixel points corresponding to internal feature points in the triangular mesh of the three-dimensional digital model according to a preset interpolation algorithm and the curvature values;
obtaining the candidate two-dimensional digital image according to the first two-dimensional pixel points and the second two-dimensional pixel points.

4. The method according to any one of claims 1-3, wherein obtaining the candidate two-dimensional digital image corresponding to the three-dimensional digital model comprises:
generating the candidate two-dimensional digital image by performing mesh parameterization based on the three-dimensional digital model.

5. The method according to any one of claims 1-4, wherein determining the candidate cervical margin line according to the candidate two-dimensional digital image comprises:
inputting the candidate two-dimensional digital image into a deep learning model pre-trained, and determining the candidate cervical margin line according to output results of the deep learning model.

6. The method according to any one of claims 1-5, before obtaining a target cervical margin line by performing error correction processing on the candidate cervical margin line in the three-dimensional digital model, the method further comprises:
determining a two-dimensional candidate cervical margin line in the candidate two-dimensional digital image to obtain a target two-dimensional digital image;
reprojecting the target two-dimensional digital image into the three-dimensional digital model, and determining the candidate cervical margin line corresponding to the two-dimensional candidate cervical margin line in the three-dimensional digital model, wherein the candidate cervical margin line is a three-dimensional candidate cervical margin line.

7. The method according to any one of claims 1-6, wherein reprojecting the target two-dimensional digital image into the three-dimensional digital model and determining the candidate cervical margin line corresponding to the two-dimensional candidate cervical margin line in the three-dimensional digital model comprises:
determining mapping relationship between two-dimensional pixel points in the target two-dimensional digital image and corresponding three-dimensional model points in the three-dimensional digital model;
determining first three-dimensional points in the three-dimensional digital model corresponding to two-dimensional points in the two-dimensional candidate cervical margin line according to the mapping relationship, and obtaining the candidate cervical margin line according to the first three-dimensional points.

8. The method according to any one of claims 1-7, wherein obtaining the target cervical margin line by performing error correction processing on the candidate cervical margin line in the three-dimensional digital model comprises:
determining a determined region and an unknown region in the three-dimensional digital model according to the candidate cervical margin line;
traversing second curvature values of sampled mesh points in the unknown region in a direction from the determined region to the unknown region according to a second preset algorithm;
determining target sampled mesh points that meet a preset convergence condition corresponding to the second preset algorithm according to the second curvature values;
obtaining the target cervical margin line by performing fitting processing on the target sampled mesh points.

9. The method according to any one of claims 1-8, wherein determining the determined region and the unknown region in the three-dimensional digital model according to the candidate cervical margin line comprises:
in the three-dimensional digital model, determining associated three-dimensional points of second three-dimensional points in the candidate cervical margin line, wherein the distance between the associated three-dimensional points and the corresponding second three-dimensional points in the candidate cervical margin line is less than a preset distance threshold;
determining a region corresponding to the second three-dimensional points and the associated three-dimensional points as the unknown region;
determining a region above the unknown region in the three-dimensional digital model as the determined region, wherein Z-axis of a coordinate system where the three-dimensional digital model of the tooth preparation is located is parallel to a tooth long axis in the three-dimensional digital model of the tooth preparation, and a direction from tooth root to tooth crown in the three-dimensional digital model of the tooth preparation is a positive direction of the Z-axis.

10. The method according to any one of claims 1-9, wherein the preset convergence condition comprises:
the second curvature value of the current sampled mesh point is greater than a curvature value of a previous adjacent adjacent sampled mesh point, and the second curvature value of the current sampled mesh point is greater than a curvature value of a next adjacent sampled mesh point.

11. The method according to any one of claims 1-10, wherein obtaining the target cervical margin line by performing fitting processing on the target sampled mesh points comprises:
obtaining a fitting curve by fitting the target sampled mesh points according to a preset fitting algorithm;
obtaining a plurality of sampled three-dimensional points by sampling the fitting curve, and obtaining the target cervical margin line according to the plurality of sampled three-dimensional points.

12. The method according to any one of claims 1-11, before traversing the second curvature values of the sampled mesh points in the unknown region of the three-dimensional digital model, the method further comprises:
identifying initial curvature values of mesh points in the unknown region, and determining candidate mesh points with initial curvature values being greater than a preset curvature threshold;
calculating product value of a preset weight and the initial curvature values of the candidate mesh points, and updating curvature values of the candidate mesh points according to the product value, wherein the preset weight is greater than 1.

13. An apparatus for extracting a cervical margin line of a tooth preparation, comprising:
a first acquisition module configured to acquire a three-dimensional digital model of the tooth preparation;
a second acquisition module configured to acquire a candidate two-dimensional digital image corresponding to the three-dimensional digital model;
a determination module configured to determine a candidate cervical margin line according to the candidate two-dimensional digital image;
an error correction module configured to obtain a target cervical margin line by performing error correction processing on the candidate cervical margin line in the three-dimensional digital model.

14. An electronic device, the electronic device comprising:
a processor;
a memory for storing executable instructions of the processor;
the processor is configured to read the executable instructions from the memory and execute the executable instructions to implement the method for extracting the cervical margin line of a tooth preparation according to any one of claims 1-12.

15. A computer-readable storage medium storing a computer program for executing the method for extracting the cervical margin line of a tooth preparation according to any one of claims 1-12.
